# EUROPÄISCHE PATENTANMELDUNG

(11) **EP 1 132 488 A1**
(43) Veröffentlichungstag der Anmeldung: **12.09.2001**
(21) Anmeldenummer: 00810192.5
(22) Anmeldetag: 07.03.2000
(51) Int. Cl.: C22B 34/12, C22B 3/00

(54) **Anlage mit Kühlkristallisatoren und Verfahren zum Betreiben der Anlage**

(71) Anmelder: Va Tech Wabag AG, 8401 Winterthur (CH)
(72) Erfinder: Moser, Hugo, 8103 Unterengstringen (CH); Ainscow, Stuart, 8400 Wintherthur (CH)
(74) Vertreter: VA TECH Patente GmbH

(57) **Zusammenfassung**

In der Anlage (1) wird mittels Kühlkristallisatoren (2) eine Produktsuspension (92) mit auskristallisierten Partikeln (96) hergestellt. Diese Suspension wird mehrstufig bei abnehmender Temperatur aus einer Mutterlösung (91) gewonnen. Dabei umfasst jede Stufe (S1, S2, ... Sn) der Anlage einen Kühlkristallisator mit einem Wärmetauscher (3), in dem aus einer Lösung (91; 94), die in der jeweiligen Stufe behandelt wird, Wärme durch gekühlte Wände (30') abgeführt wird. Die Kühlkristallisatoren bilden einen zyklischen Schaltungsverbund, der in Verbindungsleitungen (70, 71, 72, 73, 80) steuerbare Sperrorgane (61, 62, 63) enthält. Mit einer programmierten Logikschaltung (6) werden die Sperrorgane gesteuert. In einer zyklischen Abfolge gemäss einem Programm wird jeweils mindestens einer der Kühlkristallisatoren in einen Betriebszustand mit ausgeschalteter Kühlung versetzt. Während diesem Betriebszustand wird mittels der Mutterlösung (91), die beim Einspeisen in die Anlage ungesättigt ist, Wärme dem Wärmetauscher zugeführt.

## Beschreibung

Die Erfindung betrifft eine Anlage mit Kühlkristallisatoren gemäss Oberbegriff von Anspruch 1 und ein Verfahren zum Betreiben der Anlage. Ausserdem bezieht sich die Erfindung auch auf Verwendungen der Anlage.

Bei der Aufbereitung von Erzen werden Säuren verwendet, die bei einer erhöhten Temperatur Metalle aus den Erzen herauslösen. Die gelösten Metalle können aus der Säure separiert werden, indem diese abgekühlt wird. Es entstehen dabei separierbare Kristalle von Metallsalzen, die in der Regel viel Kristallwasser enthalten. Beispielsweise muss bei der Titangewinnung das Begleitmetall Eisen aus Erzen entfernt werden. Dies geschieht mit Schwefelsäure, deren Temperatur über 50°C liegt. Mittels einer mehrstufigen Kühlkristallisation lässt sich eine FeSO₄·7H₂O-Kristalle enthaltende Suspension gewinnen. Durch Sedimentation und Filtration (bei gleichzeitigem Waschen) separiert man ein Kristallisat, das 96 Gew-% des Eisensulfat-Hydrats enthält. Das Schwefelsäure enthaltende Filtrat kann wieder für den Erzaufschluss genutzt werden. Das Eisensulfat-Hydrat lässt sich als Ausgangsprodukt für eine Eisengewinnung verwenden.

Bei bekannten Kühlkristallisations-Verfahren wird die Suspension durch Entspannung abkühlt, wobei die Entspannung beispielsweise mittels Ejektoren durchgeführt wird, indem mit diesen aus der Suspension Dampf abgesaugt und der Dampf einem Kondensator zugeführt wird. Durch die Kühlung wird einerseits die Temperatur abgesenkt, wobei sich die thermodynamischen Bedingungen für eine Kristallbildung einstellen, und andererseits wird die freigesetzte Kristallisationswärme abgeführt. Da sich bei der Kristallisation die kristalline Phase nicht nur in Form von suspendierten Kristallpartikeln ausbildet, sondern da auch eine Kristallisation auf Wandungen erfolgt, müssen die an den Wandungen entstandenen Krusten periodisch unter einer Wärmezufuhr aufgelöst werden. Für die Kühlung mittels Ejektoren ist primäre Energie für eine Erzeugung von Treibdampf aufzubringen; der Energiebedarf dabei ist relativ gross.

Aufgabe der Erfindung ist es, eine Anlage mit Kühlkristallisatoren zu schaffen, für deren Betrieb weniger primäre Energie als bei dem erwähnten Verfahren benötigt wird. Diese Aufgabe wird durch die im Anspruch 1 definierte Anlage gelöst.

In der erfindungsgemässen Anlage wird mittels Kühlkristallisatoren eine Produktsuspension mit auskristallisierten Partikeln hergestellt. Diese Suspension wird mehrstufig bei abnehmender Temperatur aus einer Mutterlösung gewonnen. Dabei umfasst jede Stufe der Anlage einen Kühlkristallisator mit einem Wärmetauscher, in dem aus einer Lösung, die in der jeweiligen Stufe behandelt wird, Wärme durch gekühlte Wände abgeführt wird. Die Kühlkristallisatoren bilden einen zyklischen Schaltungsverbund, der in Verbindungsleitungen steuerbare Sperrorgane enthält. Mit einer programmierten Logikschaltung werden die Sperrorgane gesteuert. In einer zyklischen Abfolge gemäss einem Programm wird jeweils mindestens einer der Kühlkristallisatoren in einen Betriebszustand mit ausgeschalteter Kühlung versetzt. Während diesem Betriebszustand wird mittels der Mutterlösung, die beim Einspeisen in die Anlage ungesättigt ist, Wärme dem Wärmetauscher zugeführt.

Gekühlt wird in der erfindungsgemässen Anlage mittels Wärmetauschern, mit denen die Kristallisationswärme direkt aus dem Prozess abgeführt wird, also nicht indirekt wie bei den bekannten Verfahren, bei denen die Kühlung durch Entspannung durchgeführt wird. Für das erfindungsgemässe Verfahren ist daher der Energiebedarf geringer. Ausserdem besteht ein weiterer Vorteil: Es handelt sich um ein kontinuierliches Verfahren, bei dem anders als bei bekannten Verfahren nicht die gesamte Anlage periodisch in einen unproduktiven Betriebszustand gebracht werden muss, in welchem die an den Wandungen gebildeten Krusten abgeschmolzen werden.

Die abhängigen Ansprüche 2 bis 8 betreffen vorteilhafte Ausführungsformen der erfindungsgemässen Anlage. Ein Verfahren zu Betreiben der erfindungsgemässen Anlage und Verwendungen sind jeweils Gegenstand der Ansprüche 9 und 10.

Nachfolgend wird die Erfindung anhand der Zeichnungen erläutert. Es zeigen:
- Fig. 1: eine schematische Darstellung eines Gesamtprozesses, in dem die erfindungsgemässe Anlage für einen Teilprozess eingesetzt ist,
- Fig. 2: ein Schema zu einem Kühlkristallisator,
- Fig. 3: eine Ausführungsform des Kühlkristallisators der Fig. 2,
- Fig. 4: die erfindungsgemässe Anlage in einer augenfälligen Darstellung eines zyklischen Schaltungsverbunds,
- Fig. 5: ein Netz von Verbindungsleitungen, die zwischen Wärmetauschern der Anlage und zwei Wärmesenken angeordnet sind,
- Fig. 6: eine Illustration zum Kühlvorgang im Wärmetauscher der erfindungsgemässen Anlage und
- Fig. 7: einen Kristallisator, in den über eine zusätzliche Leitung Kristalle in den Prozess rückführbar sind.

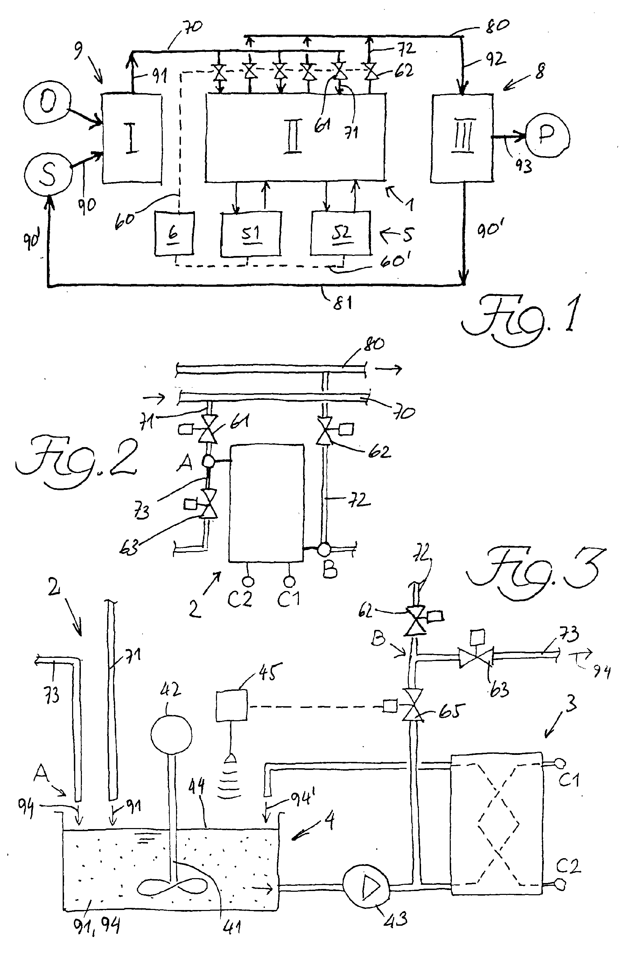

Der in Fig. 1 dargestellte Prozess findet in Anlagen 9, 1 sowie 8 statt; er umfasst den Anlagen entsprechend drei Teilprozesse I, II, III:
1.) eine Behandlung eines Erzes in der Anlage 9, der Teilprozess I, bei dem Erz aus einem Vorrat O mit einer Säure 90 aus einem Vorrat S behandelt wird, wobei beispielsweise das Erz Eisen enthält und die Säure 90 eine Schwefelsäure enthaltende Lösung ist;
2.) eine Kristallerzeugung in der Anlage 1, der Teilprozess II, in dem mehrstufig mit Kühlkristallisatoren bei abnehmender Temperatur eine Mutterlösung 91 behandelt und dabei eine Produktsuspension 92 mit auskristallisierten Partikeln hergestellt wird; und
3.) eine Separation, der Teilprozess III, bei dem aus der Suspension 92 ein Filtrat 90' von einem Kristallisat 93 getrennt wird, wobei das aus den erzeugten Kristallen, FeSO₄·7H₂O, gebildete Kristallisat 93 als Produkt P den Prozess verlässt, und das Filtrat 90' durch eine Leitung 81 in den Säurevorrat S rückgeführt wird.

Die Mutterlösung 91 gelangt über eine Verteilleitung 70 in einen von mehreren Kristallisatoren. Absperrorgane 61 in Zuführungsleitungen 71 sind bis auf eines geschlossen. Wegführungsleitungen 72 mit Absperrorganen 62 erlauben den Austritt der Produktsuspension aus einem weiteren der Kristallisatoren, der die letzte Stufe des Kristallisationsprozesses II bildet. Die Kristallisatoren bilden einen zyklischen Schaltungsverbund. Die in Verbindungsleitungen angeordneten Sperrorgane 61, 62 lassen sich mit einer programmierten Logikschaltung 6 steuern. Sie werden gemäss einem Programm in einer zyklischen Abfolge geöffnet oder geschlossen. Wärmetauscher der Kristallisatoren sind mit Wärmesenken 5 verbindbar, wobei insbesondere eine erste Wärmesenke 51 ein natürliches Gewässer wie beispielsweise ein Fluss ist, und eine zweite Wärmesenke 52 eine Kälte erzeugende Maschine ist. Die Kühlung der Kristallisatoren erfolgt nach dem Programm für die zyklische Abfolge, wobei die Steuerung auch mittels der Logikschaltung 6 durchgeführt wird. Die Steuersignale werden über Verbindungen 60 und 60' zwischen der Logikschaltung 6 und den Sperrorganen 61, 62 bzw. Wärmesenken 51, 52 übertragen.

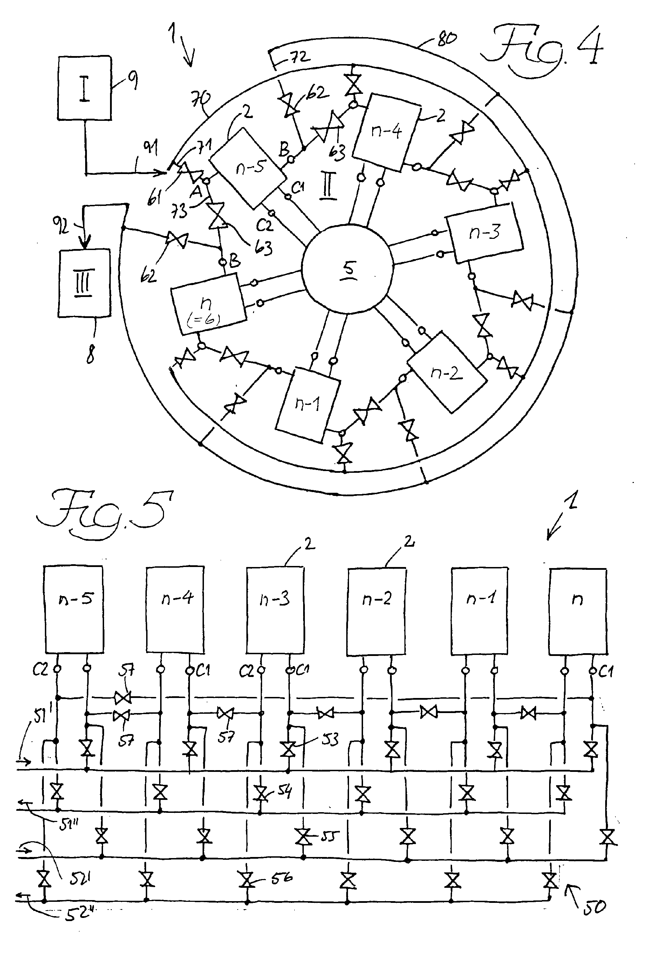

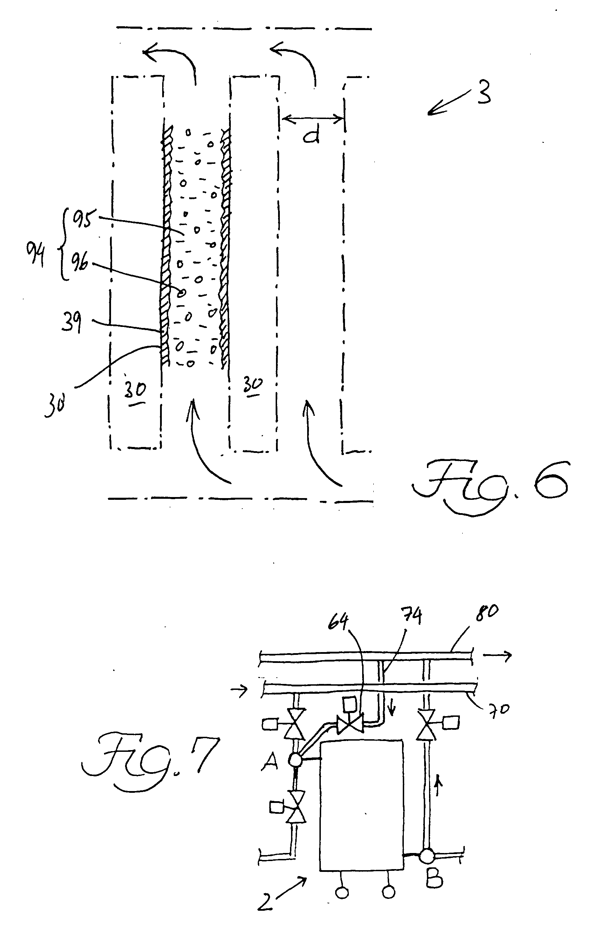

Der in Fig. 2 als Schema dargestellte Kühlkristallisator 2, in dem eine Stufe des Kristallisationsprozesses II stattfindet, ist in Fig. 3 als eine konkrete Ausführungsform abgebildet: Der Kühlkristallisator 2 ist mit einem Kreislauf ausgestattet, in dem die Mutterlösung 91 oder eine Suspension 94 mit einer Pumpe 43 umgewälzt wird. Der Kreislauf verbindet einen Wärmetauscher 3, in dem Wärme aus der Suspension 94 (oder Mutterlösung 91) durch gekühlte Wände abgeführt wird, mit einem Tank 4, in den gekühlte Suspension 94' zugeführt wird. Der Wärmetauscher 3 ist über einen Eingang C1 und einen Ausgang C2 an Wärmesenken 5 angeschlossen (siehe Fig. 4 und 5). Der Tank 4 weist ein Rührwerk 41 mit einem Antriebsmotor 42 zum Rühren des Tankinhalts 91 bzw.94 auf. Das Niveau 44 des Tankinhalts 91 bzw.94 wird mit einem Messgerät 45 (Distanzmessung mit Ultraschall) und einem Ventil 65 geregelt. Durch die Leitung 71 kann Mutterlösung 91 in den Tank 4 zugespeist werden. Die Leitungen 73 verbinden aufeinanderfolgende Stufen. Mit einem Sperrorgan 63 kann diese Verbindung unterbrochen werden. Je nach Betriebszustand des Kristallisators 2 ist entweder das Sperrorgan 63 oder das Sperrorgan 62 offen, während jeweils das andere geschlossen ist. Bei offenem Sperrorgan 62 wird die Produktsuspension 92 (der letzten Verfahrensstufe) über die Leitung 80 der Separationsanlage 8 zugeführt.

Die Pole A und B im Schema der Fig. 2 entsprechen Stellen A und B, auf die in der Fig. 3 mit Pfeilen gezeigt ist. Die Pole C1 und C2 sind die Anschlüsse an die Wärmesenken 5.

Die Figuren 4 und 5 zeigen den zyklischen Schaltungsverbund der erfindungsgemässen Anlage 1 mit n = 6 Kühlkristallisatoren 2. Die Darstellung in Fig. 4 ist so gestaltet, dass die zyklische Symmetrie gut ersichtlich wird. Die lineare Anordnung in Fig. 5 ist äquivalent zur kreissymmetrischen Anordnung der Fig. 4. Es sind alle die in Fig. 4 gezeigten Komponenten bereits aus der Beschreibung der Figuren 1 bis 3 bekannt. In Fig. 4 ist der Anschluss an die Wärmesenken 5 lediglich grob angegeben. Ein relativ komplexes Netz 50 von Verbindungsleitungen, die zwischen den Wärmetauschern 3 der Anlage 1 und zwei Wärmesenken 51 und 52 angeordnet sind, zeigt die Fig. 5. Die Wärmesenken 51 und 52 stellen beispielsweise Kühlwasser mit Temperaturen von 20°C bzw. 4°C zur Verfügung. Die Pfeile 51' und 51" geben den Zufluss von Kühlwasser aus der Wärmesenke 51 bzw. den Wegfluss des erwärmten Kühlwassers an. Entsprechendes gilt für die Pfeile 52' und 52" bezüglich der Wärmesenke 52. Mit Sperrorganen 53 und 54 lässt sich der Zu- bzw. Wegfluss von oder zur Wärmesenke 51 steuern. Entsprechendes gilt für Sperrorgane 55 und 56 bezüglich der Wärmesenke 52.

Durch Öffnen von Sperrorganen 57 lassen sich die Wärmetauscher 3 benachbarter Kristallisatoren 2 in Serie schalten, so dass das erwärmte Kühlwasser des einen Kristallisators, nämlich einer Stufe n' (n' = 2, 3, ... 6), als Kühlmedium im benachbarten Kristallisator der Stufe n'-1 weiter nutzbar ist. (In der ersten Stufe, n' = 1, wird nicht gekühlt; siehe unten). Die behandelte Mutterlösung 91 oder Produktsuspension 94 wird in der Gegenrichtung von der Stufe n'-1 zur Stufe n' gefördert. Die Sperrorgane 53 bis 57 werden so gesteuert, dass die beiden Wärmesenken 51 und 52 mit den zugeordneten Wärmetauschern 3 jeweils einen Kreislauf bilden, der vom anderen getrennt ist.

Der Betrieb einer erfindungsgemässen Anlage 1 mit n Kristallisatoren 2 (n = 6) wird nun für einen stationären Gesamtbetrieb beschrieben: Den Kristallisatoren 2 seien die Nummern n-m, mit m = 0, 1, ... n-1 zugeordnet, die nachfolgend die Bezugsnummer 2 ersetzen. Die Betriebszustände der einzelnen Kristallisatoren n-m werden periodisch durch Umschalten der Sperrorgane geändert. Eine Periode des Betriebs beginnt mit dem Umschalten der Sperrorgane. Beim Übergang einer Periode zur folgenden wechselt ein Kristallisator n-M von der höchsten Stufe Sn (= S6, im vorliegenden Beispiel mit n = 6) zur niedrigsten Stufe S1.

Auf der Stufe Sn ist während der eben abgeschlossenen Periode bei der tiefsten Betriebstemperatur auskristallisiert worden. Kristallkrusten auf den Kühlplatten des Wärmetauschers 3 haben dabei eine maximale Dicke angenommen. Beim Übergang zur Stufe S1 wird die Kühlung im Kristallisator n-M ausgeschaltet. Anschliessend werden die Kristallkrusten mit Mutterlösung 91 abgeschmolzen, deren Temperatur höher als eine Sättigungstemperatur ist. Diese Sättigungstemperatur ist die Temperatur, bei welcher sich die Lösung bezüglich der auszukristallisierenden Substanzen in einem gesättigten Zustand befindet. Bei Fortsetzung der Kühlung setzt im gesättigten Zustand die Kristallisation ein.

Die Mutterlösung 91 wird auf der Stufe S1 nicht bis zur Sättigungstemperatur abgekühlt. Die teilweise abgekühlte Mutterlösung 91 wird von der Stufe S1 zur Stufe S2 des nachfolgenden Kristallisators n-M+1 gefördert, in dem die Kühlung auf dem höchsten Temperturniveau stattfindet. Dort wird die Mutterlösung 91 bis zur Sättigungstemperatur abgekühlt. Anschliessend setzt die Kristallbildung auf der Stufe S3 im folgenden Kristallisator n-M+2 bei einem weiter abgesenkten Temperturniveau ein.

Auf der Stufe S3 beginnt sich die Produktsuspension 92 auszubilden. Sie gelangt dann Stufe für Stufe bei absinkender Temperatur unter Anwachsen des Kristallanteils bis auf die höchste Stufe Sn aus der eine Suspension 92 mit rund 10 Volumenprozent an auskristallisierten Partikeln aus der Anlage 1 abgezogen werden kann.

Die erfindungsgemässe Anlage 1 kann mehr als sechs Kristallisatoren 2 aufweisen; auch eine kleinere Anzahl ist möglich. Umfasst sie eine grosse Anzahl an Kristallisatoren 2, so kann es vorteilhaft sein, wenn die Abschmelzstufe, in der Kristallkrusten entfernt werden, in zwei oder mehr hintereinander geschalteten Kristallisatoren 2 durchgeführt wird.

Die Kristallkrusten können auch beschleunigt entfernt werden, wenn sich die Wärmetauscher 3 mit einer Wärmequelle verbinden lassen. Mit einer solchen Wärmequelle, die auf gleiche Weise wie die Wärmesenken 51 bzw 52 an die Kristallisatoren 2 angeschlossen werden, müssen sich Kühlplatten 30 auf eine Temperatur erwärmen lassen, bei der die auf den Kühlplatten 30 gebildeten Kristalle abschmelzbar sind.

Anhand der Fig. 6 soll der Kühlvorgang im Wärmetauscher 3 des Kristallisators 2 erläutert werden. Der Wärmetauscher 3 ist ein Plattenwärmetauscher mit parallel und in gleichen Abständen d angeordneten Kühlplatten 30, die in Fig. 6 lediglich strichpunktiert angedeutet sind. Auf den Oberflächen 30' der Kühlplatten 30 bilden sich aus der Produktsuspension 94 eine heterogene Kruste 39 aus, die aus Kristallkörnern und einer wässerigen Phase zwischen diesen Körnern besteht. Die feste Phase weist ein relativ langsames Kristallwachstum auf (dies ist eine Voraussetzung für die Realisierung des erfindungsgemässen Verfahrens). Daher geht die flüssige Phase 95 der Produktsuspension 94 in einen unterkühlten Zustand über, aufgrund dessen die suspendierten Kristallpartikel 96 ebenfalls wachsen können. Die unterkühlte Produktsuspension 94 wird in den Tank 4 (Fig. 3) gefördert, wo die Temperatur der Phase 95 durch die Kristallisationswärme, die durch die weiter wachsenden Kristallpartikel 96 freigesetzt wird, anwächst und sich einem Wert eines thermodynamischen Gleichgewichts nähert.

Im Plattenwärmetauscher 3 müssen die Kristallpartikel 96 durch die Zwischenräume der Kühlplatten 30 transportiert werden können, ohne dass es zu Verstopfungen kommt. Daher muss der Abstand d zwischen benachbarten Kühlplatten 30 wesentlich grösser als ein mittlerer Durchmesser der Kristallpartikel 96 sein. Ausserdem muss selbsverständlich die Dicke der Kruste 39 relativ dünn bleiben. Wenn die Zwischenräume der Kühlplatten 30 von der Produktsuspension 94 mit grosser Geschwindigkeit und turbulent durchströmt werden, erfolgt durch die mitbewegten Kristallpartikel 96 eine Erosion der Krustenoberfläche und ein beschleunigter Temperaturausgleich zwischen der Krustenoberfläche und der Phase 95. Dank dieser Einwirkung reduziert sich das Dickenwachstum der Kruste 39.

Ein kritischer Punkt des Kristallisationsverfahrens ist die Ausbildung neuer Kristalle. Mittels einer Rückspeisung von Kristallen kann die Kristallisation angeimpft werden. Zwecks Animpfung kann die Sammelleitung 80 - siehe Fig. 7 - mit absperrbaren Leitungen 74 (Absperrorgan 64) mit den Kristallisatoren 2 verbunden werden.

Die erfindungsgemässe Anlage 1 kann zur Behandlung einer Mutterlösung 91 verwendet werden, die einen organischen oder anorganischen Stoff gelöst enthält, dessen Löslichkeitskurve mit abnehmender Temperatur steil abfällt. Für einen derartigen Stoff können folgende Beispiele - mit den englischen Bezeichnungen - genannt werden:
- Oxalic Acid HO₂C·CO₂H·2H₂O
- Citric Acid C₃H₄(OH)(CO₂H)₃
- Thio Urea NH₂·CS·NH₂
- Pentaerythiol C(CH₂OH)₄
- Potassium Nitrate KNO₃
- Iron Sulphate, Copperas FeSO₄·7H₂O
- Nickel Sulphate NiSO₄·7H₂O
- Copper Sulphate CuSO₄·5H₂O
- Zinc Sulphate ZnSO₄·6H₂O oder ZnSO₄·7H₂O
- Sodium Sulphate Na₂SO₄·10H₂O.

## Patentansprüche

1. Anlage (1) mit Kühlkristallisatoren (2), in der mehrstufig bei abnehmender Temperatur aus einer Mutterlösung (91) eine Produktsuspension (92) mit auskristallisierten Partikeln (96) herstellbar ist, **dadurch gekennzeichnet**,
dass jede Stufe (S1, S2, ... Sn) einen Kühlkristallisator mit einem Wärmetauscher (3) umfasst, in dem aus einer Lösung (91; 94), die in der jeweiligen Stufe behandelt wird, Wärme durch gekühlte Wände (30') abführbar ist, dass die Kühlkristallisatoren einen zyklischen Schaltungsverbund bilden, der in Verbindungsleitungen (70, 71, 72, 73, 80) steuerbare Sperrorgane (61, 62, 63) enthält, dass mit einer programmierten Logikschaltung (6) die Sperrorgane steuerbar sind und
dass in einer zyklischen Abfolge gemäss einem Programm jeweils mindestens einer der Kühlkristallisatoren in einen Betriebszustand mit ausgeschalteter Kühlung versetzbar ist, während welchem mittels der Mutterlösung (91), die beim Einspeisen in die Anlage ungesättigt ist, Wärme dem Wärmetauscher zuführbar ist.

2. Anlage nach Anspruch 1, **dadurch gekennzeichnet**, dass der Wärmetauscher (3) ein Plattenwärmetauscher mit parallel und in gleichen Abständen (d) angeordneten Kühlplatten (30) ist und dass der Abstand zwischen benachbarten Kühlplatten wesentlich grösser als ein mittlerer Durchmesser der in der Produktsuspension (92) enthaltenen Kristallpartikel (96) ist.

3. Anlage nach Anspruch 1 oder 2, **dadurch gekennzeichnet**, dass jeder Kühlkristallisator (2) einen direkten Nachfolger hat, in den bei geöffnetem Zustand eines Sperrorgans (63) ein Teil der behandelten Lösung (91; 94), die - mit Ausnahmen - Kristallpartikel (96) enthält, kontinuierlich weitertransportierbar ist, wobei die genannten Ausnahmen durch den oder die ungekühlten Kristallisatoren und deren nachfolgender Kristallisator gegeben sind, in welchen Kristallisatoren die ungesättigte Mutterlösung (91) abgekühlt wird, schliesslich bis zu einem Zustand abgekühlt, für den die Lösung bezüglich dem auszukristallisierenden Stoff angenähert gesättigt ist.

4. Anlage nach einem der Ansprüche 1 bis 3, **dadurch gekennzeichnet**, dass die Wärmetauscher (3) mit zwei verschiedenen Wärmesenken (51, 52) verbindbar sind, wobei insbesondere die eine Wärmesenke (51) ein natürliches Gewässer wie beispielsweise ein Fluss ist und die andere Wärmesenke (52) eine Kälte erzeugende Maschine ist, die über einen Kreislauf an den Wärmetauscher anschliessbar ist.

5. Anlage nach einem der Ansprüche 1 bis 4, **dadurch gekennzeichnet**, dass die Wärmetauscher (3) mit einer Wärmequelle verbindbar sind, mit der die Kühlplatten (30) auf eine Temperatur erwärmbar sind, bei der auf den Kühlplatten gebildete Kristalle (39) abschmelzbar sind.

6. Anlage nach einem der Ansprüche 1 bis 5, **dadurch gekennzeichnet**, dass die Kühlkristallisatoren (2) jeweils mit einem pumpenbetriebenen Kreislauf (4, 43, 3) ausgestattet sind, der den Wärmetauscher (3) mit einem Tank (4) verbindet, und dass jeder Tank ein Rührwerk (41) zum Rühren seines Inhalts (91; 94) aufweist.

7. Anlage nach Anspruch 6, **dadurch gekennzeichnet**, dass an jedem Tank (4) eine Niveauüberwachung (45) des Tankinhalts angeordnet ist, mit der ein Weitertransport der Lösung (91; 94) in einen nachfolgenden Kristallisator (2) regelbar ist.

8. Anlage nach einem der Ansprüche 1 bis 7, **dadurch gekennzeichnet**, dass eine Sammelleitung (80) für die Produktsuspension mit absperrbaren Leitungen (74) mit den Kristallisatoren (2) verbunden sind, über die eine Rückspeisung von Kristallen zur Animpfung der Kristallisation durchführbar ist.

9. Verfahren zum Betreiben einer Anlage (1) gemäss einem der Ansprüche 1 bis 8, **dadurch gekennzeichnet**, dass alle n Kristallisatoren (2), die Wärmetauscher (3) enthalten, sich in verschiedenen Betriebszuständen S1, S2,... Sn befinden, denen eine Folge von absteigenden Temperaturniveaus zugeordnet ist, dass diese Betriebszustände periodisch geändert werden, wobei der Kristallisator mit dem Zustand Sn in den Zustand S1 wechselt, in welchem Zustand S1 Kristallkrusten an Wandungen (30') mit Mutterlösung (91) abgeschmolzen werden, und die weiteren Kristallisatoren in den Zustand mit dem nächsttieferen Temperaturniveau übergehen.

10. Verwendung einer Anlage (1) gemäss einem der Ansprüche 1 bis 8, **dadurch gekennzeichnet**, dass eine Mutterlösung (91) behandelt wird, die einen organischen oder anorganischen Stoff gelöst enthält, dessen Löslichkeitskurve mit abnehmender Temperatur steil abfällt, und dass für einen derartigen Stoff folgende Beispiele - mit den englischen Bezeichnungen - genannt werden können:
- Oxalic Acid HO₂C·CO₂H·2H₂O
- Citric Acid C₃H₄(OH)(CO₂H)₃
- Thio Urea NH₂·CS·NH₂
- Pentaerythiol C(CH₂OH)₄
- Potassium Nitrate KNO₃
- Iron Sulphate, Copperas FeSO₄·7H₂O
- Nickel Sulphate NiSO₄·7H₂O
- Copper Sulphate CuSO₄·5H₂O
- Zinc Sulphate ZnSO₄·6H₂O oder ZnSO₄·7H₂O
- Sodium Sulphate Na₂SO₄·10H₂O
